# EUROPÄISCHE PATENTANMELDUNG

(11) **EP 0 754 689 A1**
(43) Veröffentlichungstag der Anmeldung: **22.01.1997**
(21) Anmeldenummer: 96110964.2
(22) Anmeldetag: 08.07.1996
(51) Int. Cl.: C07D 323/06

(54) **Verfahren zur Abtrennung von Trioxan eines wässrigen Gemisches**

(30) Priorität: 19.07.1995 DE 19526307
(71) Anmelder: HOECHST AKTIENGESELLSCHAFT, 65929 Frankfurt am Main (DE)
(72) Erfinder: Arnold, Dieter, Dl., 61462 Königstein (DE); Hierholzer, Bernhard, Dr., 60385 Frankfurt (DE); Mück, Karl-Friedrich, Dr., 65207 Wiesbaden (DE); Reiss, Monika, Dl., 65824 Schwalbach (DE); Richter, Peter, Dl., 65529 Waldems-Bermbach (DE); Schnabel, Hans-Dietmar, Dl., 65817 Eppstein (DE); Wloch, Hubert, Dl., 65527 Niedernhausen (DE)

(57) **Zusammenfassung**

Die Erfindung betrifft ein Verfahren zur Abtrennung von Trioxan aus einem flüssigen Gemisch enthaltend Trioxan, Wasser und Formaldehyd, dadurch gekennzeichnet, daß man das flüssige Gemisch in einer ersten Destillationsstufe bei einem niedrigen Druck destilliert, das anfallende Destillat in einer zweiten Destillationsstufe bei einem höheren Druck destilliert und Trioxan als Sumpfprodukt abnimmt.

## Beschreibung

Die Erfindung betrifft ein Verfahren zur Herstellung von Trioxan aus einem flüssigen Gemisch enthaltend, Trioxan, Wasser und Formaldehyd.

Großtechnisch (nach Ullmann, Band A 11 (1988), S. 645) wird Trioxan in einem mehrstufigen Prozeß produziert, bei dem Formaldehyd in wäßriger Lösung mit Katalysatorhilfe trimerisiert und das erhaltene Reaktionsgemisch aufgearbeitet wird. Das Gemisch aus Trioxan, Wasser und Formaldehyd, das auch Nebenprodukte enthält, wird mit Methylenchlorid oder einem anderem mit Wasser nicht mischbaren Lösungsmittel wie Ethylenchlorid oder Benzol extrahiert. In einer nachfolgenden Destillation wird das Lösungsmittel zurückgewonnen und der Extraktivdestillation wieder zugeführt. Dieses Verfahren benötigt große Mengen Lösungsmittel, die mit hohem Energieaufwand zurückgewonnen werden müssen. Anfallende Emissionen müssen aufwendig entsorgt werden, da Methylenchlorid und Benzol als gefährliche Schadstoffe eingestuft sind.

Der Erfindung lag daher die Aufgabe zugrunde, ein Verfahren bereitzustellen, das ohne Lösungsmittel auskommt.

Erfindungsgemäß wird die Aufgabe dadurch gelöst, daß man das flüssige Gemisch in einer ersten Destillationsstufe bei einem niedrigen Druck destilliert, das anfallende Destillat in einer zweiten Destillationsstufe bei einem höheren Druck destilliert und Trioxan als Sumpfprodukt abnimmt.

Gegenstand der Erfindung ist somit ein Verfahren zur Abtrennung von Trioxan aus einem flüssigen Gemisch enthaltend Trioxan, Wasser und Formaldehyd, dadurch gekennzeichnet, daß man das flüssige Gemisch in einer ersten Destillationsstufe bei einem niedrigen Druck destilliert, das anfallende Destillat in einer zweiten Destillationsstufe bei einem höheren Druck destilliert und Trioxan als Sumpfprodukt abnimmt. Hierbei wird das ternäre Azeotrop Formaldehyd, Trioxan, Wasser Überwunden und es gelingt, nahezu reines Trioxan zu gewinnen.

Das Verfahren wird vorzugsweise bei Absolutdrücken von 0,1 bis 20 bar durchgeführt. Bevorzugt ist der Bereich von 0,2 bis 10 bar, besonders bevorzugt ist ein niedriger Druck im Bereich von 0,1 bis 2 bar und ein höherer Druck im Bereich von 2 bis 20 bar und ganz besonders günstig ist ein niedriger Druck von etwa 0,5 bar und ein höherer Druck von etwa 6 bar.

Die Erfindung wurde ermöglicht, durch die überraschende Entdeckung, daß das ternäre Azeotrop Formaldehyd/Trioxan/Wasser durch Druckerhöhung in einer im Sinne der Erfindung günstigen Weise verschoben wird.

Gegenstand der Erfindung ist weiter ein Verfahren zur Herstellung von Trioxan, bei dem man Formaldehyd in wäßriger Lösung mit Katalysatorhilfe trimerisiert und das erhaltene flüssige Gemisch aufarbeitet, dadurch gekennzeichnet, daß man das flüssige Gemisch in einer ersten Destillationsstufe bei einem niedrigen Druck destilliert, das anfallende Destillat in einer zweiten Destillationsstufe bei einem höheren Druck destilliert und Trioxan als Sumpfprodukt abnimmt.

Die Vorteile der erfindungsgemäßen Verfahren sind im wesentlichen darin zu sehen, daß kein Lösungsmittel mehr erforderlich ist, somit das Emissionsproblem gelöst ist und die gesamte Verfahrensführung vereinfacht wird, bei einer erheblichen Reduzierung der Betriebs- und Investitionskosten. Das erhaltene Trioxan eignet sich als Desinfektionsmittel und zur Herstellung von Polyoxymethylenen oder mehrwertigen Alkoholen.

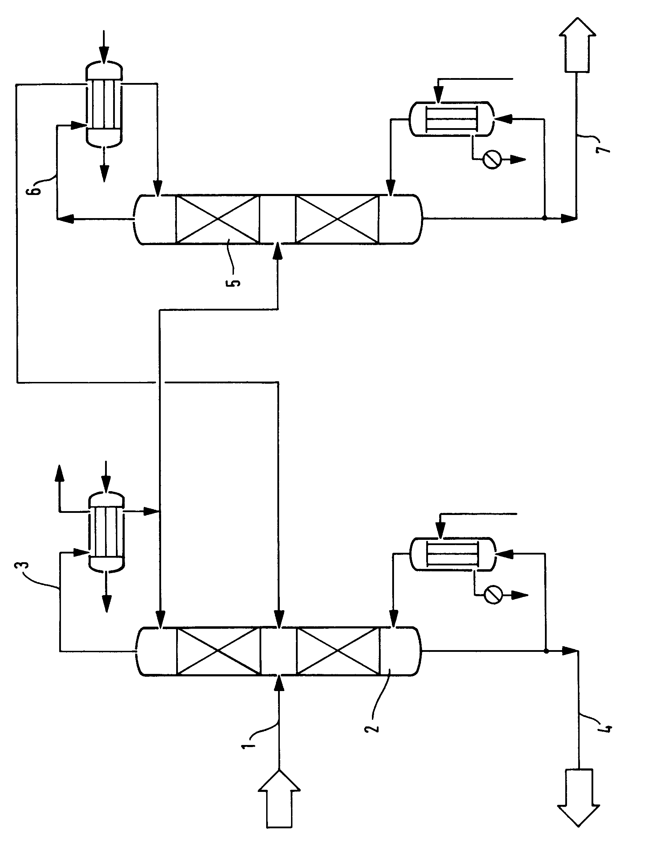

Im folgenden wird das erfindungsgemäße Verfahren entsprechend Anspruch 1 anhand einer möglichen Ausführungsform, die in der Figur als Fließbild dargestellt ist, näher erläutert.

Ein flüssiges Gemisch 1 enthaltend Trioxan, Wasser und Formaldehyd wird einer Destillationskolonne 2 vorzugsweise im mittleren Bereich zugeführt und bei einem niedrigen Druck in ein Destillat 3 und ein Sumpfprodukt 4 zerlegt, das an Trioxan verarmt ist. In einer nachgeschalteten Kolonne 5, deren mittlerer Bereich mit dem Kopf der Kolonne 2 verbunden ist, wird das Destillat 3 aus der Kolonne 2 bei einem höheren Druck in ein Destillat 6 und ein Sumpfprodukt 7, das im wesentlichen aus reinem Trioxan besteht, aufgetrennt. Das an Trioxan verarmte Destillat 6 wird in die Kolonne 2 zurückgeführt, und zwar vorzugsweise in deren mittleren Bereich.

Das Verfahren wird in dem folgenden Beispiel weiter verdeutlicht.

In einer Anlage entsprechend dem Fließbild wurde ein Strom eines wäßrigen Gemisches 1, im wesentlichen bestehend aus 20 % (Gew.) Trioxan (TOX), 50 % Formaldehyd (FOH) und 30 % Wasser (H₂O) behandelt. Der niedrige Druck in der Kolonne 2 betrug etwa 0,5 bar, der höhere Druck in der Kolonne 5 etwa 6 bar. Eine Analyse der einzelnen Ströme ergab folgende Konzentrationen (Gew.-%):

| | TOX | FOH | H₂O | Bemerkung |
|---|---|---|---|---|
| Destillat 3 | 72,0 | 6,3 | 21,7 | ternäres Azeotrop bei 0,5 bar |
| Sumpfprodukt 4 | 0,2 | 59,1 | 40,7 | |
| Destillat 6 | 60 | 9,2 | 30,8 | ternäres Azeotrop bei 6 bar |
| Sumpfprodukt 7 | ∼ 100 % | | | nahezu reines TOX |

## Patentansprüche

1. Verfahren zur Abtrennung von Trioxan aus einem flüssigen Gemisch enthaltend Trioxan, Wasser und Formaldehyd, dadurch gekennzeichnet, daß man das flüssige Gemisch in einer ersten Destillationsstufe bei einem niedrigen Druck destilliert, das anfallende Destillat in einer zweiten Destillationsstufe bei einem höheren Druck destilliert und Trioxan als Sumpfprodukt abnimmt.

2. Verfahren nach Anspruch 1, dadurch gekennzeichnet, daß der niedrige oder der höhere Druck im Bereich von 0,1 bis 20 bar (abs) liegt.

3. Verfahren nach Anspruch 1, dadurch gekennzeichnet, daß der niedrige oder der höhere Druck im Bereich von 0,2 bis 10 bar (abs) liegt.

4. Verfahren nach Anspruch 1, dadurch gekennzeichnet, daß der niedrige Druck im Bereich von 0,1 bis 2 bar (abs) liegt.

5. Verfahren nach Anspruch 1, dadurch gekennzeichnet, daß der höhere Druck im Bereich von 2 bis 20 bar (abs) liegt.

6. Verfahren nach Anspruch 1, dadurch gekennzeichnet, daß der niedrige Druck bei etwa 0,5 bar (abs) liegt.

7. Verfahren nach Anspruch 1, dadurch gekennzeichnet, daß der höhere Druck bei etwa 6 bar (abs) liegt.

8. Verfahren zur Herstellung von Trioxan, bei dem man Formaldehyd in wäßriger Lösung mit Katalysatorhilfe trimerisiert und das erhaltene flüssige Gemisch aufarbeitet, dadurch gekennzeichnet, daß man das flüssige Gemisch in einer ersten Destillationsstufe bei einem niedrigen Druck destilliert, das anfallende Destillat in einer zweiten Destillationsstufe bei einem höheren Druck destilliert und Trioxan als Sumpfprodukt abnimmt.

9. Verwendung von Trioxan, erhalten aus einem Verfahren gemäß Anspruch 1 oder 8 als Desinfektionsmittel oder zur Herstellung von Polyoxymethylenen oder mehrwertigen Alkoholen.

10. Vorrichtung zur Durchführung des Verfahrens nach Anspruch 1, bei der zwei Destillationskolonnen hintereinander geschaltet sind, dadurch gekennzeichnet, daß der Kopf der ersten Destillationskolonne mit dem mittleren Bereich der zweiten Destillationskolonne verbunden ist und daß der Kopf der zweiten Destillationskolonne mit dem mittleren Bereich der ersten Destillationskolonne verbunden ist.
